(19) Europäisches Patentamt
European Patent Office
Office européen des brevets

(11) Publication number: 0 097 888 B1

(12) **EUROPEAN PATENT SPECIFICATION**

(45) Date of publication of patent specification: **26.02.86**

(51) Int. Cl.[4]: **C 12 P 17/18** // C12N11/14 ,(C12P17/18, C12R1:465)

(21) Application number: **83105921.7**

(22) Date of filing: **16.06.83**

(54) **Process for producing thienamycin employing Streptromyces cattleya cells immobilized to kieselguhr.**

(30) Priority: **25.06.82 US 392060**

(43) Date of publication of application: **11.01.84 Bulletin 84/02**

(45) Publication of the grant of the patent: **26.02.86 Bulletin 86/09**

(84) Designated Contracting States: **CH DE FR GB IT LI NL**

(56) References cited:
GB-A-2 058 776
US-A-4 335 212

(73) Proprietor: **MERCK & CO. INC.**
**126, East Lincoln Avenue P.O. Box 2000**
**Rahway New Jersey 07065 (US)**

(72) Inventor: **Buckland, Barry C.**
**626 Boulevard**
**Westfield New Jersey 07090 (US)**
Inventor: **Baker, Edward E.**
**70 Green Street**
**Woodbridge New Jersey 07095 (US)**
Inventor: **Prevoznak, Richard**
**202 Jersey Street**
**South Amboy New Jersey 08879 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

EP 0 097 888 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The instant invention relates to a new and useful process for preparing the antibiotic, thienamycin. More particularly, the instant invention relates to a process for thienamycin wherein cells of a thienamycin producing microorganism are immobilized on kieselguhr and the cells so immobilized are employed in the production of thienamycin by fermentation techniques.

The antibiotic, thienamycin, is a uniquely structured β-lactam compound of the formula:

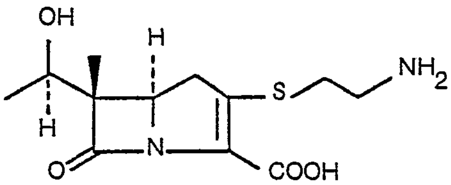

As will be noted, thienamycin is structurally characterized by having the unique trans β-configuration at the hydroxyethyl group at the 6-position. Thienamycin may be obtained as a natural product by fermentation of a thienamycin producing strain of *Streptomyces cattleya* by techniques already fully described, such as, for example, United States Patent No. 3,950,357, issued April 13, 1976, wherein its antibiotic activity in animal and human therapy and in inanimate systems also is disclosed.

Preparation of thienamycin by classical fermentation techniques employing a thienamycin producing strain of *Streptomyces cattleya*, such as, for example, *Streptomyces cattleya* NRRL 8057 (Northern Regional Research Laboratories, Northern Utilization Research and Development Division, Agricultural Research Service, U.S. Department of Agriculture, Peoria, Ill., as disclosed in the above-identified U.S. patent, although successfully producing the antibiotic, never-the-less presents certain difficulties inherent in such classical fermentation processes. Included among these are relatively low productivity due in part to feedback inhibition phenomenon and degradation of product due to complex media composition used in cell growth. Further, the high level of impurities in the fermentation broth resulting from the complex media employed for cell growth makes isolation and purification of the thienamycin produced difficult and expensive. Also, since optimum conditions for growth of cells may be quite different from those required for product formation, selection of appropriate media and conditions for production of product becomes complex and difficult. The process of the instant invention eliminates or materially decreases those difficulties.

The instant invention is based upon applicants' discovery that cells of a thienamycin producing strain of *Streptomyces cattleya* can be immobilized to kieselguhr and employed in the production of thienamycin by fermentation techniques with significant advantages over classical fermentation techniques. The cells of *Streptomyces cattleya* are immobilized by growing in submerged culture in the presence of kieselguhr. Kieselguhr is composed essentially of fossilized shells of diatoms produced amorphous silica and is characterized by a high internal pore volume. During growth of *Streptomyces cattleya* in the presence of kieselguhr the mycelium of the microorganism grows into the pores of the kieselguhr and becomes tightly attached to the kieselguhr. The cells so immobilized may be maintained in a productive state by intermittent decanting and feeding of a production medium throughout a prolonged period extending for as long as 190 days.

Applicants have found that thienamycin production as described above results in significant advantages as compared to classical fermentation techniques. Notably, the process of the instant invention separates the growth stage of the cells from the production stage thus allowing conditions to be optimized for product formation with consequent increase in overall productivity. The process allows for extended productive operation, as long as 190 days, as opposed to 5 days for conventional batch fermentation techniques. Intermittent or continuous removal of product and replenishment of production medium decreases feedback inhibition and product degradation. Also, the product stream, being largely freed from suspended cellular material and unused nutrients, is much purer and final product separation and purification is less difficult and cheaper.

Production of thienamycin according to the process of this invention may be carried out as follows:

Kieselguhr preparation

Commercially available kieselguhr qualities such as Johns-Manville celite®, for example, Celite® 560 (Johns-Manville, New York, New York), are treated to eliminate fines by floatation. The kieselguhr is suspended in water, agitated vigorously and allowed to settle for ten seconds, then decanted. This process is repeated several times until the kieselguhr settles to a clear supernatant within ten seconds. The kieselguhr is then dried to a constant weight and is used without further treatment in preparing immobilized cells. Desirably the kieselguhr will have a particle diameter of from 100 to 900 micrometer.

Inoculum development

The stock culture of the producing microorganism may be maintained either in the lyophilized state or as frozen vegetative mycelium. The inoculum for production is developed by suspending the appropriate stock culture in sterile seed medium and incubating at 28—34°C for 1 to 18 hours (typical seed media are illustrated in the appended Examples). Conveniently, the incubation is carried out on a rotary shaker with a two inch throw rotating at 220 r.p.m.

Growth and immobilization

Growth as well as production may be carried out at any volume depending upon production

requirements. Typically, for small scale runs, fermentation is carried out in submerged culture in 250 ml non-baffled Erlenmeyer flasks containing 20.0 ml. of a suitable growth medium (illustrated in the appended Examples) and 3.0 g of kieselguhr. The growth medium is sterilized by conventional procedures at 120°C for 15 minutes. Growth is initiated by introducing a measured quantity of the inoculum described above. After inoculation, the culture is incubated for 24 to 96 hours at 28 to 37°C on a rotary shaker with a two inch throw rotating at 220 r.p.m. During the growth stage, the mycelia grow into the pores of the kieselguhr and is entrapped thus becoming immobilized in the kieselguhr matrix.

Production stage

The immobilized cells are harvested by decanting the spent growth medium. An appropriate production medium (illustrated in the appended Examples) then is added to the immobilized cells and the culture is incubated at 28—37°C for 24 hours. The product, which is water soluble, is contained in the medium fraction and is harvested by decanting and fresh production medium is added to the immobilized cells to replace the volume decanted. Harvest of product is continued on a periodic basis usually from 4 to 24 hours. This can be done on an intermittant basis. Where harvest is conducted on a continuous basis, addition of fresh media and removal of spent media conveniently is adjusted so as to effect a complete turnover of reactor volume in a 4—24 hour period. Duration and productivity of the immobilized cells will vary with the production medium. Immobilized cultures prepared as described above have been shown to produce for periods exceeding 190 days. The course of the fermentation is monitored by pH measurement and by product accumulation.

Isolation of product from the decanted medium is accomplished by techniques already fully described in the art. Such techniques form no part of the instant invention.

The media employed in the growth and production stages described above may vary widely in composition. In general, however, the antibiotic is produced during the aerobic fermentation of the producing microorganism in aqueous nutrient media. Aqueous media, such as those employed for the production of other antibiotics, are suitable for producing thienamycin. Such media contain sources of carbon, nitrogen and inorganic salts assimilable by the microorganism.

Typically, carbohydrates such as sugars, for example, glucose, fructose, maltose, sucrose, xylose, mannitol and the like and starches such as grain, for example, oats, rye, cornstarch, corn meal and the like can be used, either alone or in combination, as sources of assimilable carbon in the nutrient medium. The exact quality of carbohydrate utilized in the medium depends in part on the nature of the other components of the medium, but, in general, the amount of carbohydrate usually varies between about 0.1% to about 7% by weight of the medium.

Many proteinaceous materials may be used as nitrogen sources in the fermentation. Suitable nitrogen sources will include, for example, yeast hydrolysates, primary yeast, soy bean meal, cottonseed flour, hydrolysates of casein, cornsteep liquor, distiller's solubles, tomato paste and the like. The nitrogen sources, either alone or in combination, are used in amounts ranging from about 0.005% to about 6% by weight of the medium.

Among the nutrient inorganic salts which can be employed in the media are the customary salts capable of yielding sodium, potassium, ammonium, calcium, phosphate, sulfate, chloride, carbonate and the like ions. Also trace metals may be included such as, for example, cobalt, manganese, iron, magnesium and the like. Such media, generally, will display a pH ranging from about 6 to about 8.

The best mode contemplated by applicants for carrying out their invention is further illustrated in the following working Examples. No limitation, however, is intended except as defined in the appended claims.

Example 1

A Stage—Stock Culture: *Streptomyces cattleya* NRRL 8057

1. Vessel: 2 ml. glass vial containing 2.0 ml frozen vegetative mycelium
2. Medium: same as Example 2
3. Inoculum: see as Example 2
4. Incubation: same as Example 2
5. Storage: same as Example 2

B Stage—Same as Example 2

C Stage—Same as Example 2

D Stage—(Growth): same as Example 2

E Stage—(Production)

1. Vessel: same as Example 2
2. Medium: 100 ml production medium

| | g./liter |
|---|---|
| Acid hydrolized casein—(vitamin free—salt free) | 0.460 |
| Sodium lactate (60%) | 2.4 |
| Primary yeast (Yeast Products, Inc.—Clifton, N.J.) | 1.0 |
| $KH_2PO_4$ | 0.250 |
| $MgSO_4 \cdot 7H_2O$ | 0.0625 |
| $CaCl_2$ (anh.) | 0.0425 |

| | g./liter |
|---|---|
| $FeSO_4 \cdot 7H_2O$ | 0.00125 |
| $CuSO_4 \cdot 5H_2O$ | 0.000125 |
| $MnCl_2 \cdot 4H_2O$ | 0.000125 |
| $CoCl_2 \cdot 6H_2O$ | 0.001 |
| MES Buffer | 14.6 |
| pH 6.5 | |

3. Inoculum: same as Example 2
4. Incubation: same as Example 2
5. Harvest: same as Example 2
6. Productivity/ml. kieselguhr (Celite)/Day: 34.5 μg
7. Duration of Productivity: 6 days
8. Production medium changed (day 6) to 100 ml of: same as Example 2
9. Incubation: same as Example 2
10. Harvest: same as Example 2
11. Productivity/ml. kieselguhr (Celite)/Day: 55.8 μg
12. Duration of Productivity: 8 days

Example 2
Thienamycin production
A Stage—Stock Culture: *Streptomyces Cattleya*
1. Vessel: 2 ml. glass vial containing 2.0 ml frozen vegetative mycelium
2. Medium:

| | g./liter |
|---|---|
| Sucrose | 30.0 |
| Distillers solubles (B & F) | 15.0 |
| Ardamine® YEP (Yeast Products, Inc., Clifton, N.J.) | 5.0 |
| corn gluten meal | 5.0 |
| Distilled water | |
| pH 7.5 (with NaOH) | |

3. Inoculum: 2 ml. of 24 hour culture
4. Incubation: none
5. Storage: −80°C indefinitely

B Stage
1. Vessel: 250 ml. 3-baffled Erlenmeyer flask
2. Medium: 50 ml. seed medium

| | g./liter |
|---|---|
| Primary yeast | 10.0 |
| Ardamine YEP (Yeast Products, Inc., Clifton, N.J.) | 10.0 |
| Cerelose | 10.0 |
| Tap water | |
| pH 7.0 | |

3. Inoculum: 1.0 ml. A Stage
4. Incubation: 28°C for 24 hours on rotary shaker with two-inch throw at 220 r.p.m.

C Stage
1. Vessel: 250 ml. 3 baffled Erlenmeyer flask
2. Medium: 50 ml. seed medium

| | g./liter |
|---|---|
| Mono sodium glutamate $H_2O$ | 5.0 |
| $NH_4Cl$ | 1.5 |
| $K_2HPO_4$ | 2.0 |
| Inositol | 0.4 |
| NaCl | 0.5 |
| $MgSO_4 \cdot 7H_2O$ | 0.5 |
| $FeSO_4 \cdot 7H_2O$ | 0.025 |
| $ZnSO_4 \cdot 7H_2O$ | 0.010 |
| Paraamino benzoic acid | 0.0001 |
| $CoCl_2 \cdot 6H_2O$ | 0.010 |
| Glycerin | 10.0 |
| Ardamine YEP | 15.0 |
| Distilled water | |
| pH 7.0 | |
| Then add $CaCO_3$ | 0.250 |

3. Inoculum: 2.0 ml. B Stage
4. Incubation: same as B Stage

D State—(Growth)
1. Vessel: 250 ml. no-baffled Erlenmeyer flask
2. Medium 20 ml. growth medium+3.0 g kieselguhr (Celite)

| | g./liter |
|---|---|
| Mono sodium glutamate · $H_2O$ | 3.75 |
| L-Isoleucine | 2.4 |
| $NH_4Cl$ | 0.75 |
| $K_2HPO_4$ | 1.0 |

| | g./liter |
|---|---|
| $CoCl_2 \cdot 6H_2O$ | 0.010 |
| $MgSO_4 \cdot 7H_2O$ | 0.5 |
| $FeSO_4 \cdot 7H_2O$ | 0.025 |
| Glycerin | 10.0 |
| MES Buffer (2-[N-morpholino] ethanesulfonic acid)—(Sigma Chemical Co., St. Louis, Mo.) | 14.6 |
| Distilled water | |
| pH 7.0 | |

3. Inoculum: 2 ml. C Stage
4. Incubation: 28°C for 72 hours on rotary shaker with two inch throw at 220 r.p.m.

E Stage—(Production)
1. Vessel: 250 ml. non-baffled Erlenmeyer flask
2. Medium: 100 ml. production medium

| | g./liter |
|---|---|
| Mono sodium glutamate · $H_2O$ | 0.920 |
| Sodium lactate (60%) | 2.4 |
| L-Isoleucine | 0.590 |
| $KH_2PO_4$ | 0.500 |
| $MgSO_4 \cdot 7H_2O$ | 0.125 |
| $CaCl_2$ (anhyd.) | 0.085 |
| $FeSO_4 \cdot 7H_2O$ | 0.0025 |
| $CuSO_4 \cdot 5H_2O$ | 0.00025 |
| $MnCl_2 \cdot 4H_2O$ | 0.00025 |
| $CoCl_2 \cdot 6H_2O$ | 0.001 |
| MES Buffer | 14.6 |
| Distilled water | |
| pH 6.5 | |

3. Inoculum: immobilized cells from D Stage
4. Incubation: 34°C for 24 hours on rotary shaker with two inch throw at 220 r.p.m.
5. Harvest: harvesting daily by decanting spent medium containing thienamycin
6. Productivity/ml. kieselguhr (Celite)/Day: 130 µg
7. Duration of Productivity: 14 days

Example 3
Thienamycin production

A Stage—Stock Culture: *Streptomyces Cattleya*
1. Vessel: 2 ml. glass vial containing 2.0 ml. frozen vegetative mycelium
2. Medium: same as Example 2
3. Inoculation: 2.0 ml. of 24 hour culture
4. Incubation: none
5. Storage: −80°C indefinitely

B Stage
1. Vessel: 250 ml. 3 baffled Erlenmeyer flask
2. Medium: same as Example 2 (C Stage)
3. Inoculum: 1.0 ml. A Stage
4. Incubation: 28°C for 24 hours on rotary shaker with two inch throw at 220 r.p.m.

C Stage—(Growth)
1. Vessel: 250 ml. non-baffled Erlenmeyer flask
2. Medium: 20 ml. growth medium+3.0 g. kieselguhr (Celite)

| | g./liter |
|---|---|
| Corn steep liquor | 5.0 |
| Tomato paste | 40.0 |
| Oat flour | 10.0 |
| Glucose | 10.0 |
| Trace element #2* | 10.0 |
| pH 6.8 | |

3. Inoculum: 2.0 ml. B Stage
4. Incubation: 72 hours at 28°C on rotary shaker with two inch throw at 220 r.p.m.

D Stage—(Production)
1. Vessel: 250 ml. non-baffled Erlenmeyer flask
2. Medium: same as Example 2
3. Inoculum: immobilized cells from C Stage
4. Incubation: 28°C for 24 hours or rotary shaker with two inch throw at 220 r.p.m.
5. Harvest: same as Example 2 (E Stage)
6. Productivity/ml. kieselguhr (Celite)/Day: 130 µg
7. Duration of Productivity: 14 days

*Trace element #2

| | mg./liter |
|---|---|
| $FeSO_4 \cdot 7H_2O$ | 10.0 |
| $MnSO_4 \cdot 4H_2O$ | 10.0 |
| $CuCl_2 \cdot 2H_2O$ | 0.25 |
| $CaCl_2$ | 1.0 |

| | mg./liter |
|---|---|
| $H_3BO_3$ | 0.56 |
| $(NH_4)_6MoO_2 \cdot 4H_2O$ | 0.19 |
| $ZnSO_4 \cdot 7H_2O$ | 2.0 |

Example 4
Thienamycin production

A Stage—same as Example 3

B Stage—same as Example 3

C Stage—(Growth)
1. Vessel: same as Example 3.
2. Medium: 20 ml. growth medium+3.0 g. kieselguhr (Celite)

| | g./liter |
|---|---|
| Yeast extract | 10.0 |
| Dextrose | 10.0 |
| Agar | 15.0 |
| pH 7.0 | |

3. Inoculum: 2.0 ml B Stage
4. Incubation: same as Example 3

D Stage—(Production)
1. Vessel: same as Example 3
2. Medium: same as Example 3
3. Inoculum: same as Example 3
4. Incubation: same as Example 3
5. Harvest: same as Example 3
6. Productivity/ml. kieselguhr (Celite)/Day: 120 μg
7. Duration of Productivity: 14 days

Example 5
Thienamycin production

A Stage—same as Example 3

B Stage—same as Example 3

C Stage—(Growth)
1. Vessel: same as Example 3
2. Medium: 20.0 ml BACTO® Nutrient Broth (Difco Laboratories, Detroit, Mich.) +3.0 g. Celite
3. Inoculum: same as Example 3
4. Incubation: same as Example 3

D Stage—(Production)
1. Vessel: same as Example 3
2. Medium: same as Example 3
3. Inoculum: same as Example 3
4. Incubation: same as Example 3
5. Harvest: same as Example 2
6. Productivity/ml kieselguhr (Celite)/Day: 120 μg
7. Duration of Productivity: 14 days

Example 6
Thienamycin production

A Stage—same as Example 2

B Stage—same as Example 2 (C Stage)

C Stage—(Growth) same as Example 2

D Stage—(Production)
1. Vessel: same as Example 2
2. Medium: 100.0 ml production medium

| | g./liter |
|---|---|
| Sodium lactate (60%) | 2.4 |
| Ardamine pH (Yeast Products, Inc., Clifton, N.J.) | 2.0 |
| MES Buffer | 14.6 |
| Distilled water | |
| pH 6.5 | |

3. Inoculum: same as Example 2
4. Incubation: same as Example 2
5. Harvest: same as Example 2
6. Productivity/ml. kieselguhr (Celite)/Day: 60 μg
7. Duration of Productivity: 33 days

Example 7
A Stage—same as Example 2

B Stage—same as Example 2

D Stage—(Growth)—same as Example 2

E Stage—(Production)
1. Vessel: same as Example 2
2. Medium: 100 ml production medium

| | g./liter |
|---|---|
| Primary yeast | 2.0 |
| Sodium lactate (60%) | 2.4 |
| MES Buffer | 14.6 |
| Distilled water | |
| pH 6.5 | |

3. Inoculum: same as Example 2
4. Incubation: same as Example 2
5. Harvest: same as Example 2
6. Productivity/ml. kieselguhr (Celite/Day: 88.7 μg
7. Duration of Productivity: 32 days

8. Production medium changed (day 32) to 100 ml of:

| | g./liter |
|---|---|
| Ardamine pH | 2.0 |
| Sodium lactate (60%) | 2.4 |
| MES Buffer | 14.6 |
| Distilled water | |
| pH 6.5 | |

9. Incubation: same as Example 2
10. Harvest: same as Example 2
11. Productivity/ml. kieselguhr (Celite)/Day: 53.6 μg
12. Duration of Productivity: 14 days
13. Production medium changed (day 46) to 100 ml of:

| | g./liter |
|---|---|
| Acid hydrolyzed casein—(vitamin free—salt free) | 0.92 |
| Sodium lactate (60%) | 2.4 |
| $KH_2PO_4$ | 2.0 |
| $MgSO_4 \cdot 7H_2O$ | 0.5 |
| $CaCl_2$ (anh.) | 0.34 |
| $FeSo_4 \cdot 7H_2O$ | 0.01 |
| $CuSO_4 \cdot 5H_2O$ | 0.001 |
| $MnCl_2 \cdot 4H_2O$ | 0.001 |
| $CoCl_2 \cdot 6H_2O$ | 0.01 |
| MES Buffer | 14.6 |
| Distilled water | |
| pH 6.5 | |

14. Incubation: same as Example 2
15. Harvest: same as Example 2
16. Productivity/ml. kieselguhr (Celite)/Day: 68.3 μg
17. Duration of Productivity: 8 days
18. Production medium changed (day 54) to 100 ml of:

| | g./liter |
|---|---|
| NZ Amine type E (Humko—Sheffield—Norwich, N.Y.) | 0.920 |
| Sodium lactate (60%) | 2.4 |
| $KH_2PO_4$ | 0.5 |
| $MgSO_4 \cdot 7H_2O$ | 0.125 |
| $CaCl_2$ (anh.) | 0.085 |
| $FeSO_4 \cdot 7H_2O$ | 0.0025 |
| $CuSO_4 \cdot 5H_2O$ | 0.00025 |
| $MnCl_2 \cdot 4H_2O$ | 0.00025 |
| $CoCl_2 \cdot 6H_2O$ | 0.010 |
| MES Buffer | 14.6 |
| Distilled water | |
| pH 6.5 | |

19. Incubation: same as Example 2
20. Harvest: same as Example 2
21. Productivity/ml. kieselguhr (Celite)/Day: 27.0 μg
22. Duration of Productivity: 8 days
23. Production medium changed (day 62) to 100 ml of: same as Step 2
24. Incubation: same as Example 2
25. Harvest: same as Example 2
26. Productivity/ml. kieselguhr (Celite)/Day: 69.0 μg
27. Duration of Productivity: 25 days
28. Production medium changed (day 86) to 100 ml of:

| | g./liter |
|---|---|
| Acid hydrolyzed casein—(vitamin free—salt free) | 0.460 |
| Sodium lactate (60%) | 2.4 |
| Primary yeast | 1.0 |
| $KH_2PO_4$ | 0.250 |
| $MgSO_4 \cdot 7H_2O$ | 0.0625 |
| $CaCl_2$ (anh.) | 0.425 |
| $FeSO_4 \cdot 7H_2O$ | 0.00125 |
| $CuSO_4 \cdot 5H_2O$ | 0.000125 |
| $CoCl_2 \cdot 6H_2O$ | 0.005 |
| $MnCl_2 \cdot 4H_2O$ | 0.000125 |
| MES Buffer | 14.6 |
| Distilled water | |
| pH 6.5 | |

29. Incubation: same as Example 2
30. Harvest: same as Example 2
31. Productivity/ml. kieselguhr (Celite)/Day: 37.2 µg
32. Duration of Productivity: 11 days
33. Production medium changed (day 98) to MES buffer without nutrient supplement
34. Incubation: same as Example 2
35. Harvest: same as Example 2
36. Productivity/ml. kieselguhr (Celite)/Day: 0 after 4 days
37. Duration of Productivity: 13 days
38. Production medium changed (day 111) to 100 ml of: same as Step 28
39. Incubation: same as Example 2
40. Harvest: same as Example 2
41. Productivity/ml. kieselguhr (Celite)/Day: increased over a five day period to 24.7 µg
42. Duration of Productivity: 3 days
43. Production medium changed (day 119) to 100 ml of:

| | g./liter |
|---|---|
| Monosodium glutamate | 0.920 |
| L-Isoleucine | 0.590 |
| Sodium lactate (60%) | 2.4 |
| $KH_2PO_4$ | 2.0 |
| $MgSO_4 \cdot 7H_2O$ | 0.5 |
| $CaCl_2$ (anh.) | 0.34 |
| $FeSO_4 \cdot 7H_2O$ | 0.01 |
| $CuSO_4 \cdot 5H_2O$ | 0.001 |
| $MnCl_2 \cdot 4H_2O$ | 0.001 |
| $CoCl_2 \cdot 6H_2O$ | 0.01 |
| MES Buffer | 14.6 |
| Distilled water | |
| pH 6.5 | |

44. Incubation: same as Example 2
45. Harvest: same as Example 2
46. Productivity/ml. kieselguhr (Celite)/Day: 77 µg
47. Duration of Productivity: 25 days
48. Production medium changed (day 144) to 100 ml of:

| | g./liter |
|---|---|
| Acid hydrolyzed casein—(vitamin free—salt free) | 0.460 |
| Sodium lactate (60%) | 2.4 |
| Primary yeast | 1.0 |
| $KH_2PO_4$ | 0.250 |
| $MgSO_4 \cdot 7H_2O$ | 0.0625 |
| $CaCl_2$ (anh.) | 0.0425 |
| $FeSO_4 \cdot 7H_2O$ | 0.00125 |
| $CuSO_4 \cdot 5H_2O$ | 0.000125 |
| $CoCl_2 \cdot 6H_2O$ | 0.001 |
| $MnCl_2 \cdot 4H_2O$ | 0.000125 |
| MES Buffer | 14.6 |
| pH 6.5 | |

49. Incubation: same as Example 2
50. Harvest: same as Example 2
51. Productivity/ml. kieselguhr (Celite)/Day: 50.0 µg
52. Duration of Productivity: 21 days
53. Production medium changed (day 165) to 100 ml of:

| | g./liter |
|---|---|
| Monosodium glutamate | 0.920 |
| L-Isoleucine | 0.590 |
| Sodium lactate (60%) | 2.4 |
| $KH_2PO_4$ | 2.0 |
| $MgSO_4 \cdot 7H_2O$ | 0.5 |
| $CaCl_2$ (anh.) | 0.34 |
| $FeSO_4 \cdot 7H_2O$ | 0.01 |
| $CuSO_4 \cdot 5H_2O$ | 0.001 |
| $MnCl_2 \cdot 4H_2O$ | 0.001 |
| $CoCl_2 \cdot 6H_2O$ | 0.001 |
| MES Buffer | |
| Distilled water | |
| pH 6.5 | |

54. Incubation: same as Example 2
55. Harvest: same as Example 2
56. Productivity/ml. kieselguhr (Celite)/Day: 72.9 µg
57. Duration of Productivity: 15 day 1
58. Production medium changed (day 180) to 100 ml of MES buffer without nutrient supplement

59. Incubation: same as Example 2
60. Harvest: same as Example 2
61. Productivity/ml. kieselguhr (Celite)/Day: decreased to 0 over a three day period
62. Duration of Productivity: 4 days
63. Production medium changed (day 187) to 100 ml of: same as Step 53
64. Incubation: same as Example 2
65. Harvest: same as Example 2
66. Productivit/ml. kieselguhr (Celite)/Day: 60.1 µg
67. Duration of Productivity: 5 days
68. Discontinued on day 193

**Claims**

1. A process for producing thienamycin by immobilized cell fermentation which comprises:

(a) growing *Streptomyces cattleya* in submerged culture in a nutrient medium in the presence of kieselguhr until the mycelia of the growing cells grows into the kieselguhr pores;

(b) separating the immobilized cells so produced from the growth medium;

(c) maintaining the immobilized cells in a productive state in the presence of a production nutrient medium; and

(d) withdrawing a portion of the production medium containing produced thienamycin and replacing the withdrawn medium with an equivalent quantity of fresh production medium.

2. The process of Claim 1 wherein the kieselguhr has a particle diameter between 100 and 900 micrometer.

3. The process of Claim 2 wherein the withdrawal and replacement of production media is made on a 4—24 hour cycle.

4. The process of Claim 3 wherein the withdrawal is intermittent.

5. The process of Claim 3 wherein the withdrawal is continuous.

**Patentansprüche**

1. Ein Verfahren zur Herstellung von Thienamycin durch Fermentation immobilisierter Zellen, welches umfasst:

(a) das Züchten von Streptomyces cattleya in Submerskultur in einem Nährmedium in Anwesenheit von Kieselgur, bis das Myzel der wachsenden Zellen in die Kieselgur-poren einwächst;

(b) Abtrennen der so erzeugten immobilisierten Zellen von dem Züchtungsmedium;

(c) Halten der immobilisierten Zellen in einem produktiven Zustand, in Anwesenheit eines Produktions-Nährmediums; und

(d) Entnahme eines Teils des Produktionsmediums, das erzeugtes Thienamycin enthält, und Ersatz des entnommenen Mediums durch eine äquivalente Menge von frischem Produktionsmedium.

2. Das Verfahren nach Anspruch 1, bei dem das Kieselgur einen Teilchendurchmesser zwischen 100 und 900 Mikrometer hat.

3. Das Verfahren nach Anspruch 2, bei dem die Entnahme und der Ersatz der Produktionsmedien in einem 4—24 h Zyklus erfolgt.

4. Das Verfahren nach Anspruch 3, bei dem die Entnahme intermittierend erfolgt.

5. Das Verfahren nach Anspruch 3, bei dem die Entnahme kontinuierlich erfolgt.

**Revendications**

1. Un procédé pour la production de la thiénamycine par fermentation avec des cellules immobilisées qui comprend

(a) la culture de *Streptomyces cattleya* en culture submergée dans un milieu nutritif en présence de kieselguhr jusqu'à ce que les mycéliums des cellules en culture se développent dans les pores du kieselguhr;

(b) la séparation des cellules immobilisées ainsi produites d'avec le milieu de culture;

(c) le maintien des cellules immobilisées en un stade productif en présence d'un milieu nutritif de production; et

(d) le prélèvement d'une portion du milieu de production contenant la thiénamycine produite et le remplacement du milieu prélevé par une quantité équivalente de milieu de production frais.

2. Le procédé de la revendication 1 dans lequel le kieselguhr a un diamètre des particules entre 100 et 900 micromètres.

3. Le procédé de la revendication 2 dans lequel le prélèvement et le remplacement des milieux de production sont effectués selon un cycle de 4—24 heures.

4. Le procédé de la revendication 3 dans lequel le prélèvement est intermittent.

5. Le procédé de la revendication 3 dans lequel le prélèvement est continu.